# EUROPEAN PATENT APPLICATION

(11) **EP 4 814 130 A1**
(43) Date of publication of application: **30.09.2026**
(21) Application number: 26167454.3
(22) Date of filing: 25.03.2026
(51) Int. Cl.: C12Q 1/6844, C12N 15/10

(54) **FED-BATCH IVT ASSAY FOR NTP AND RNA ANALYSIS VIA HPLC**

(30) Priority: 25.03.2025 US 202563777399 P
(71) Applicant: Aldevron, LLC, Fargo, North Dakota 58104 (US)
(72) Inventor: FAKHRI, Mustafa, Fargo, ND 58104 (US); YOUNG, Rebecca, Fargo, ND 58104 (US); LAGACY, Ryan, Fargo, ND 58104 (US); COOPER, Jonathan William, Fargo, ND 58104 (US); PARIHAR, Aakanksha Singh, Fargo, ND 58104 (US)
(74) Representative: Maiwald GmbH

(57) **Abstract**

Methods for monitoring an in vitro transcription (IVT) process include obtaining a sample from an IVT process at a first timepoint. The sample is run on a liquid chromatography (LC) column, the LC column characterized by being a silica column with C18 bonding and a Lewis acid/Lewis base endcapping. One or more peaks are quantitated against a first calibration curve.

## Description

### INTRODUCTION

Fed-batch in vitro transcription (IVT) is a widely used technique in the production of RNA, particularly for applications like RNA vaccines, therapeutic RNA, and RNA-based diagnostics. Unlike traditional batch systems, where all the reactants are added at the beginning, the fed-batch process involves the controlled addition of reagents or substrates throughout the reaction. This approach aims to optimize the reaction by maintaining an ideal environment for the enzyme activity, which can often degrade or become less efficient over time.

Use of fed-batch systems can extend the duration of transcription reactions, enhance the yield of RNA, and improve the overall quality of the synthesized RNA. By continuously or intermittently adding nucleotides, energy sources, or other necessary components, the system helps to sustain the activity of RNA polymerase, the key enzyme involved in transcription. This method is particularly useful when producing large quantities of RNA for high-demand applications, such as mRNA vaccines, where yield and quality are crucial.

One of the key benefits of fed-batch IVT is the ability to control the reagent levels during transcription, preventing depletion or imbalance of critical components. This control also helps mitigate the accumulation of byproducts, which can inhibit the enzyme and reduce the efficiency of RNA synthesis. Moreover, the fed-batch process allows for the optimization of other parameters, such as temperature and pH, further enhancing the reaction's performance. However, careful monitoring and management are required to ensure that the feeding process is consistent and does not introduce any undesirable contaminants or variability that could affect the final RNA product.

In terms of scalability, fed-batch IVT offers significant advantages over traditional batch systems, especially when scaling up for industrial or therapeutic purposes. The increased yield and improved efficiency of the process make it a preferred choice for large-scale RNA production, where the quality and quantity of the RNA must meet stringent regulatory standards

### SUMMARY

Examples presented herein relate to a method of monitoring an in vitro transcription (IVT) process. The method includes obtaining a sample from an IVT process at a first timepoint; running the sample on a liquid chromatography (LC) column, the LC column characterized by being a silica column with C18 bonding and a Lewis acid/Lewis base endcapping; and quantitating one or more peaks against a first calibration curve.

In other examples presented herein, the LC column is further characterized by a first mobile phase and a second mobile phase. In further examples presented herein, the first mobile phase is Triethylammonium bicarbonate (TEAB). In other further examples presented herein, the first mobile phase is at a 25 mM concentration. In other examples presented herein, the second mobile phase is Acetonitrile (ACN). In further examples presented herein, the second mobile phase is ramped from a 15% initial concentration to a 100% final concentration.

In other examples presented herein, the method further includes generating the first calibration curve. In further examples presented herein, the first calibration curve is configured to have an R² value of at least 0.998 for all nucleotides.

In other examples presented herein, the sample is one of a clean sample or a crude sample. In other examples presented herein, the method further includes quantifying an RNA product by: running a DNA-only standard; determining a DNA-only concentration; and subtracting the DNA-only concentration from a mixed RNA/DNA concentration peak of the one or more peaks.

In other examples presented herein, the IVT process is one of a fed batch IVT process and a simple IVT process. In further examples presented herein, the fed batch IVT process is used, and one or more additional reagents are added to the IVT process based on at least one quantitated peak, based on at least two quantitated peaks, based on at least three quantitated peaks, based on at least four quantitated peaks, based on at least five quantitated peaks, based on at least six quantitated peaks, or based on seven quantitated peaks. In other further examples presented herein, the at least one quantitated peak is selected from a peak for CTP, a peak for ATP, a peak for UTP, a peak for GTP, a peak for RNA, a peak for plasmid DNA, and a peak for a cap analog. In other further examples presented herein, at least the peak for CTP and the peak for UTP do not overlap with each other. In still further examples presented herein, the peak for CTP, the peak for ATP, the peak for UTP, the peak for GTP, the peak for RNA and the peak for a cap analog do not overlap with any other peak. In other further examples presented herein, the one or more additional reagents added to the IVT process improve yield of RNA, decrease use of additional reagents, or both.

In other examples presented herein, high-performance liquid chromatography is used. In other examples presented herein, the method further includes obtaining a sample from the IVT process at additional timepoints; running each additional sample on a liquid chromatography (LC) column, the LC column characterized by being a silica column with C18 bonding and a Lewis acid/Lewis base endcapping; and quantitating one or more peaks against a first calibration curve. In further examples presented herein, the method further includes adding one or more additional reagents to the IVT process based on at least one quantitated peak, based on at least two quantitated peaks, based on at least three quantitated peaks, based on at least four quantitated peaks, based on at least five quantitated peaks, based on at least six quantitated peaks, or based on seven quantitated peaks. In other further examples presented herein, the at least one quantitated peak is selected from a peak for CTP, a peak for ATP, a peak for UTP, a peak for GTP, a peak for RNA, a peak for plasmid DNA, and a peak for a cap analog. In still further examples presented herein, at least the peak for CTP and the peak for UTP do not overlap with each other. In still further examples presented herein, the peak for CTP, the peak for ATP, the peak for UTP, the peak for GTP, the peak for RNA and the peak for a cap analog do not overlap with any other peak. In other examples presented herein, the one or more additional reagents added to the IVT process improve yield of RNA, decrease use of additional reagents, or both

In other examples presented herein, the quantitating one or more peaks against a first calibration curve is used to determine degradation or contamination in the IVT process.

Other examples presented herein relate to a method of in vitro transcription. The method includes generating a DNA template; initiating a transcription reaction including one or more reagents; monitoring the transcription reaction by: obtaining a sample from an IVT process at a first timepoint; running the sample on a liquid chromatography (LC) column, the LC column characterized by being a silica column with C18 bonding and a Lewis acid/Lewis base endcapping; quantifying one or more peaks against a calibration curve; and purifying product RNA.

In other examples presented herein, the LC column is further characterized by a first mobile phase and a second mobile phase. In further examples presented herein, the first mobile phase is Triethylammonium bicarbonate (TEAB). In other further examples presented herein, the first mobile phase is at a 25 mM concentration. In other examples presented herein, the second mobile phase is Acetonitrile (ACN). In further examples presented herein, the second mobile phase is ramped from a 15% initial concentration to a 100% final concentration.

In other examples presented herein, the method further includes generating the first calibration curve. In further examples presented herein, the first calibration curve is configured to have an R² value of at least 0.998 for all nucleotides.

In other examples presented herein, the sample is one of a clean sample or a crude sample. In other examples presented herein, the method further includes quantifying an RNA product by: running a DNA-only standard; determining a DNA-only concentration; and subtracting the DNA-only concentration from a mixed RNA/DNA concentration peak of the one or more peaks.

In other examples presented herein, the IVT process is one of a fed batch IVT process and a simple IVT process. In further examples presented herein, the fed batch IVT process is used, and one or more additional reagents are added to the IVT process based on at least one quantitated peak, based on at least two quantitated peaks, based on at least three quantitated peaks, based on at least four quantitated peaks, based on at least five quantitated peaks, based on at least six quantitated peaks, or based on seven quantitated peaks. In other further examples presented herein, the at least one quantitated peak is selected from a peak for CTP, a peak for ATP, a peak for UTP, a peak for GTP, a peak for RNA, a peak for plasmid DNA, and a peak for a cap analog. In other further examples presented herein, at least the peak for CTP and the peak for UTP do not overlap with each other. In still further examples presented herein, the peak for CTP, the peak for ATP, the peak for UTP, the peak for GTP, the peak for RNA and the peak for a cap analog do not overlap with any other peak. In other further examples presented herein, the one or more additional reagents added to the IVT process improve yield of RNA, decrease use of additional reagents, or both.

In other examples presented herein, high-performance liquid chromatography is used. In other examples presented herein, the method further includes obtaining a sample from the IVT process at additional timepoints; running each additional sample on a liquid chromatography (LC) column, the LC column characterized by being a silica column with C18 bonding and a Lewis acid/Lewis base endcapping; and quantitating one or more peaks against a first calibration curve. In further examples presented herein, the method further includes adding one or more additional reagents to the IVT process based on at least one quantitated peak, based on at least two quantitated peaks, based on at least three quantitated peaks, based on at least four quantitated peaks, based on at least five quantitated peaks, based on at least six quantitated peaks, or based on seven quantitated peaks. In other further examples presented herein, the at least one quantitated peak is selected from a peak for CTP, a peak for ATP, a peak for UTP, a peak for GTP, a peak for RNA, a peak for plasmid DNA, and a peak for a cap analog. In still further examples presented herein, at least the peak for CTP and the peak for UTP do not overlap with each other. In still further examples presented herein, the peak for CTP, the peak for ATP, the peak for UTP, the peak for GTP, the peak for RNA and the peak for a cap analog do not overlap with any other peak. In other examples presented herein, the one or more additional reagents added to the IVT process improve yield of RNA, decrease use of additional reagents, or both

In other examples presented herein, the quantitating one or more peaks against a first calibration curve is used to determine degradation or contamination in the IVT process.

### Definition and interpretation of selected terms

As used herein, "g" represents gram; "L" represents liter; "mg" represents "milligram (10-3 gram);" "mL" or "cc" represents milliliter (10-3 liter). One "µL" equals to one micron liter (10-6 liter). The units "g/100g," "g/100mL," or "g/L" are units of concentration or content of a component in a composition. One "mg/L" equals to one ppm (part per million). "Da" refers to Dalton, which is the unit for molecular weight; One Da equals to one g/mol. The unit of temperature used herein is degree Celsius (°C).

The term "about" is used in conjunction with numeric values to include normal variations in measurements as expected by persons skilled in the art and is understood to have the same meaning as "approximately" and to cover a typical margin of error, such as ±15%, ±10%, ±5%, ±1%, ±0.5%, or even ±0.1% of the stated value. The term "about" also encompasses amounts that differ due to different equilibrium conditions for a composition resulting from a particular initial composition. Whether or not modified by the term "about," the claims include equivalents to the quantities.

It should be noted that, as used in this specification and the appended claims, the singular forms "a," "an," and "the" include plural referents unless the content clearly dictates otherwise. Thus, for example, reference to a composition containing "a compound" includes having two or more compounds that are either the same or different from each other. It should also be noted that the term "or" is generally employed in its sense including "and/or" unless the content clearly dictates otherwise. As used herein, "and/or" refers to and encompasses any and all possible combinations of one or more of the associated listed items, as well as the lack of combinations when interpreted in the alternative ("or").

In the interest of brevity and conciseness, any ranges of values set forth in this specification contemplate all values within the range and are to be construed as support for claims reciting any sub-ranges having endpoints which are real number values within the specified range in question. By way of a hypothetical illustrative example, a disclosure in this specification of a range of from 1 to 5 shall be considered to support claims to any of the following ranges: 1-5; 1-4; 1-3; 1-2; 2-5; 2-4; 2-3; 3-5; 3-4; and 4-5.

The term "substantially" is utilized herein to represent the inherent degree of uncertainty that can be attributed to any quantitative comparison, value, measurement, or other representation. The term "substantially" is also utilized herein to represent the degree by which a quantitative representation can vary from a stated reference without resulting in a change in the basic function of the subject matter at issue.

The term "substantially free" may refer to any component that the composition of the disclosure lacks or mostly lacks. When referring to "substantially free" it is intended that the component is not intentionally added to compositions of the disclosure. Use of the term "substantially free" of a component allows for trace amounts of that component to be included in compositions of the disclosure because they are present in another component. However, it is recognized that only trace or de minimus amounts of a component will be allowed when the composition is said to be "substantially free" of that component. Moreover, if a composition is said to be "substantially free" of a component, if the component is present in trace or de minimus amounts it is understood that it will not affect the effectiveness of the composition. It is understood that if an ingredient is not expressly included herein or its possible inclusion is not stated herein, the disclosure composition may be substantially free of that ingredient. Likewise, the express inclusion of an ingredient allows for its express exclusion thereby allowing a composition to be substantially free of that expressly stated ingredient.

The term "comprise," "comprises," and "comprising" as used herein, specify the presence of the stated features, integers, steps, operations, elements, and/or components, but do not preclude the presence or addition of one or more other features, integers, steps, operations, elements, components, and/or groups thereof.

As used herein, the transitional phrase "consisting essentially of" means that the scope of a claim is to be interpreted to encompass the specified materials or steps recited in the claim and those that do not materially affect the basic and novel characteristic(s) of the claimed disclosure. Thus, the term "consisting essentially of" when used in a claim of this disclosure is not intended to be interpreted to be equivalent to "comprising."

As used herein, the terms "increase," "increasing," "increased," "enhance," "enhanced," "enhancing," and "enhancement" (and grammatical variations thereof) describe an elevation of at least about 1%, 5%, 10%, 15%, 25%, 50%, 75%, 100%, 150%, 200%, 300%, 400%, 500% or more as compared to a control.

As used herein, the terms "reduce," "reduced," "reducing," "reduction," "diminish," and "decrease" (and grammatical variations thereof), describe, for example, a decrease of at least about 1%, 5%, 10%, 15%, 20%, 25%, 35%, 50%, 75%, 80%, 85%, 90%, 95%, 97%, 98%, 99%, or 100% as compared to a control. In particular embodiments, the reduction can result in no or essentially no (i.e., an insignificant amount, e.g., less than about 10% or even 5% or even 1%) detectable activity or amount.

A variety of additional inventive aspects will be set forth in the description that follows. The inventive aspects can relate to individual features and to combinations of features. It is to be understood that both the forgoing general description and the following detailed description are exemplary and explanatory only and are not restrictive of the broad inventive concepts upon which the embodiments disclosed herein are based.

### BRIEF DESCRIPTION OF THE DRAWINGS

The accompanying drawings, which are incorporated in and constitute a part of the description, illustrate several aspects of the present disclosure. A brief description of the drawings is as follows:
FIG. 1 is a chromatogram of an IVT sample run on an HPLC column, according to embodiments of the present disclosure, demonstrating coelution of mRNA and pDNA.
FIG. 2 is a flowchart of an example method of monitoring an in vitro transcription (IVT) process.
FIG. 3 is a flowchart of an example method of in vitro transcription.
FIG. 4 is an example chromatogram with multiple runs of IVT reagents for generating NTP calibration curves.
FIG. 5 is an example graph of calibration curves generated using data from the chromatogram of FIG. 4.
FIG. 6 is an example chromatogram with multiple runs of IVT reagents for generating cap reagent and mRNA product calibration curves.
FIG. 7 is an example graph of a calibration curves generated using data from the chromatogram of FIG. 5.
FIG. 8 is an example graph of a calibration curve for mRNA.
FIG. 9 is a comparison of three gradients demonstrating an example preferred gradient for provided adequate separation in a desired timeframe.

### DETAILED DESCRIPTION

Disclosed herein are systems, methods, and processes for improved monitoring and maintenance of reagents during a fed-batch in-vitro transcription (IVT) process. In a fed-batch IVT process, reagents such as nucleotides (NTPs), energy sources (like ATP), and co-factors are added gradually throughout the transcription reaction to maintain optimal conditions for RNA synthesis. This controlled feeding helps sustain RNA polymerase activity and prevents depletion of critical components. Monitoring changes in reagent concentrations is crucial to ensure the reaction remains efficient and balanced. Regular analysis of key variables like reagent levels, pH, and temperature allows for timely adjustments, ensuring consistent RNA yield and minimizing byproduct accumulation that could inhibit the process.

Currently there is no reliable way to measure changes in consumed reagents, such as NTPs and cap analogs, and RNA product concentration to monitor the fed-batch IVT and inform feedings in real-time for an ongoing process and/or subsequent feedings of an ongoing process or a subsequent process. The methods disclosed herein enable the individual quantitation of the IVT process components, e.g., four distinct NTPs, cap analogs, and mRNA, at one or more timepoints throughout the process to drive improved process development. The disclosed methods can be effectively applied to crude IVT samples, requiring no clean up step to deliver usable results.

Embodiments of the present disclosure provide for improved measurement and monitoring of NTPs, cap reagent, mRNA, pDNA template, etc. during an IVT-process to improve feeding. Embodiments of the present disclosure relate to improvements to conventional high-performance liquid chromatography (HPLC) methods, as well as new applications of the disclosed HPLC method. The improved measurement methods and protocols disclosed herein provide for improved execution of IVT procedures generally, but may be particularly advantageous in fed-batch IVT. In fed-batch IVT, RNA is made by adding additional reagents during production to improve yield. The methods disclosed herein can quantitate reaction components at time-points to optimize amounts of reagents added over time.

In conventional methods for observing NTPs, the HPLC method typically involves using a reverse-phase column or an ion-exchange column, where nucleotides can be separated based on their size, charge, and hydrophobicity. The methods disclosed herein apply HPLC for the detection of residual NTPs in IVT reactions, ensuring that the concentration of reactants is optimized for high-efficiency RNA synthesis. Similarly, for RNA analysis, the system can separate the RNA from any byproducts, unincorporated nucleotides, or contaminants. UV detection at 260 nm is commonly used to measure RNA, as nucleic acids absorb strongly at this wavelength.

Problems and limitations arise due to the complexity of IVT samples, which contain a mix of RNA polymerase, buffers, and byproducts. This complexity can hinder the separation and quantification of NTPs and RNA products. NTPs themselves are difficult to resolve accurately due to their structural similarity which causes the peaks to overlap, a difficulty which may be particularly apparent in rapid, time-sensitive monitoring protocols which are able to be quickly executed, often at the expense of accuracy. In particular, CTP and UTP peaks are known to overlap one another. Additionally, RNA degradation or fragmentation during the process can complicate analysis, and carryover contamination from previous samples can affect results.

Methods of the present disclosure distinguish all four NTPs from one another and further distinguishes the cap reagent. In embodiments, RNA and pDNA elute as one peak that is separate from other reagents. In embodiments, the methods disclosed herein are applicable to IVT procedures, such as crude IVT, and require no clean-up step. Advantages of the presently disclosed methods include clear separation of the CTP and UTP bases, which tend to co-elute in conventional methods. Further advantages include separation of modified NTPs from traditional NTPs.

Advantageous applications of the present disclosure include measurement of residual or impurity NTP, cap analog, RNA, or linear template in places where it was supposed to be removed. Methylating enzyme activity can be assessed using the methods of the present disclosure. For example, SAM (S-adenosylmethionine) and SAH (S-adenosylhomocysteine) show up as distinct peaks using the disclosed methods. Detection of NTP degradation is possible with the methods disclosed herein as shoulder peaks may indicate degraded or di/monophosphate versions of NTPs, such that peak shape provides a quality check on reagents.

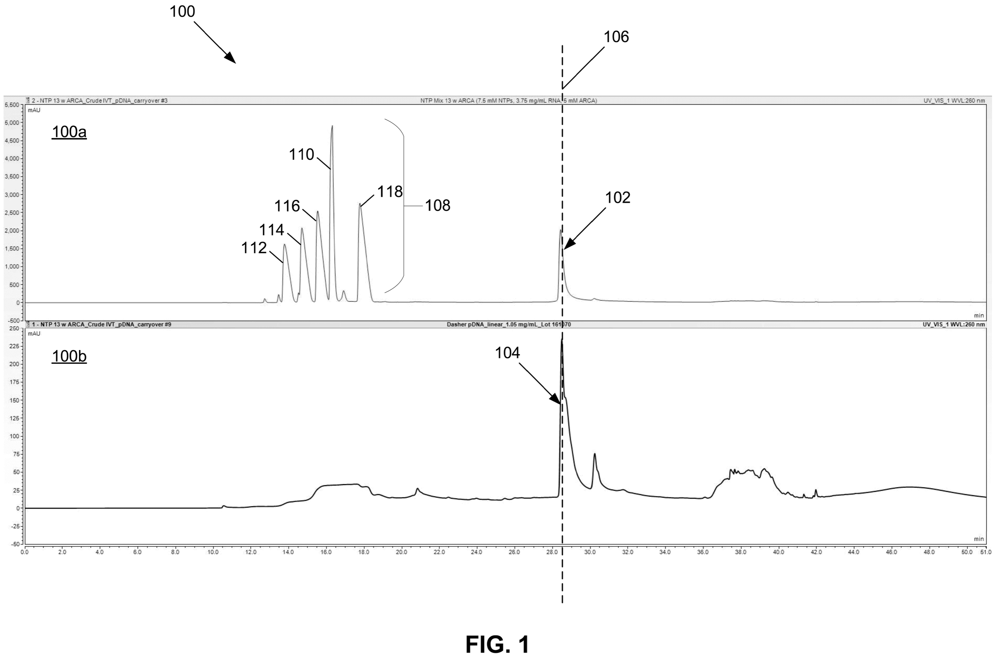

FIG. 1 is an example chromatogram 100 of an IVT sample run on an HPLC column, according to embodiments of the present disclosure, demonstrating coelution of mRNA and pDNA. A mRNA peak 102 appears at a same retention time 106 in chromatogram 100a as a pDNA peak 104 in chromatogram 100b. Also visible in chromatogram 100 are five distinct peaks which appear together at group 108. Group 108 includes a cap reagent peak 110, ARCA in this example, and separate peaks for each NTP, with a CTP peak 112, a UTP peak 114, a GTP peak 116, and an ATP peak 118. Due to the coelution of mRNA and pDNA, product RNA can be quantified by subtraction of a known amount of template DNA introduced into the process.

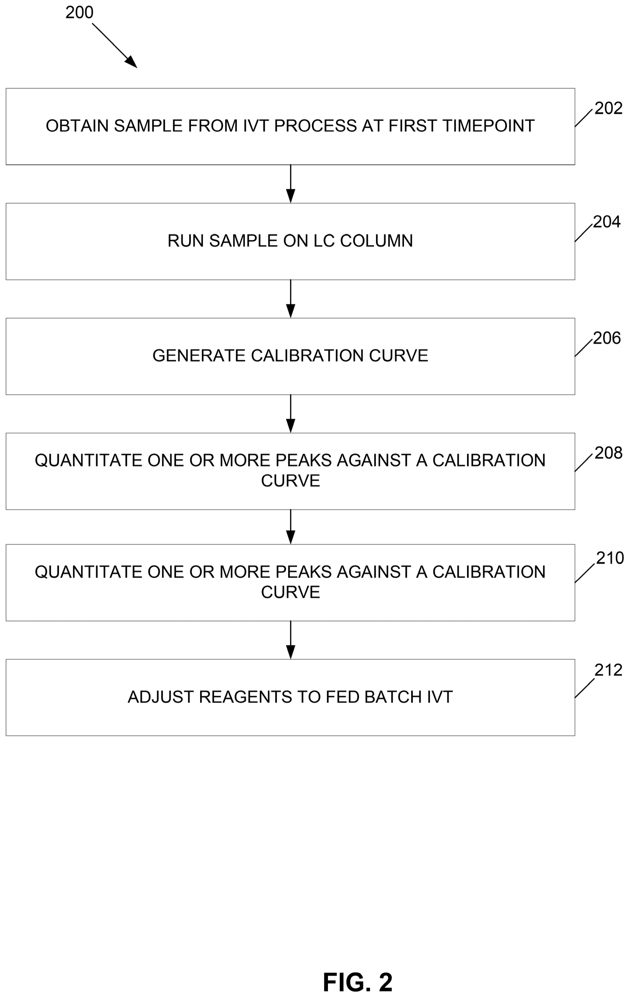

FIG. 2 is a flowchart of an example method 200 of monitoring an in vitro transcription (IVT) process. In embodiments, the IVT process a fed batch IVT process or a simple IVT process. In a simple IVT setup, all components (nucleotides, RNA polymerase, DNA template, buffer, and other required factors) are mixed together at the start of the reaction. The transcription proceeds for a defined period, and the reaction is stopped after the allotted time. Simple IVT offers advantageous simplicity, rapid setup, and cost-effectiveness.

In fed-batch IVT, reagents (e.g., NTPs, cap analogs, and other co-factors) are gradually added to the reaction over time, allowing for the reaction to continue longer, maintaining optimal conditions for RNA polymerase activity and increasing the overall yield of RNA. As discussed above, the methods of the present disclosure provide for monitoring of reagent consumption in a fed-batch IVT process and enables informed addition of additional reagents during the fed-batch process. The methods of the present disclosure, such as example method 200, also provides information which may improve a user's overall understanding of the IVT process and enable the determination of an effective predetermined timing for feedings to apply to subsequent reactions or runs of the process. In embodiments, method 200 may be applied to inform feedings in real time for an ongoing fed-batch IVT process. While many of the examples discussed herein focus on application to fed-batch IVT processes, the principles of the present disclosure are also readily application to simple IVT, and provide useful analysis of a simple IVT output which may inform subsequent IVT process design and setup.

At operation 202, a sample is obtained from an IVT process at a timepoint. In embodiments, multiple samples are obtained from the IVT process. In embodiments, some or all of the multiple samples are taken at additional timepoints.

In embodiments, the sample is a clean sample or a crude sample. A clean sample has undergone purification steps to remove impurities, leaving only the target molecule (e.g., purified RNA or a specific protein) for analysis. Techniques like gel filtration, precipitation, or further chromatography steps may be used to isolate the desired product from contaminants. In IVT, after transcription, the RNA product is typically purified to remove leftover nucleotides, enzymes, and buffer components, resulting in a cleaner sample that allows for more accurate analysis and quantification. A crude sample is one that has not undergone extensive purification. It typically contains a mix of target molecules (like RNA, proteins, or nucleotides) along with impurities such as byproducts, residual reagents, enzymes, and other contaminants from the experimental process. In the case of in vitro transcription (IVT), a crude sample may contain, for example, unincorporated nucleotides, IVT enzymes (e.g., RNA polymerase, RNase inhibitor, pyrophosphatase), buffer components, and degradation products. Despite some challenges associated with crude samples, as the impurities can interfere with the detection of the target molecules and complicate analysis by producing additional peaks or noise in the chromatogram, the ability to quickly and effectively analyze a crude sample may be advantageous for evaluation of an ongoing IVT process.

At operation 204, the sample is run on a liquid chromatography (LC) column. In embodiments where multiple samples are drawn, for instance across multiple timepoints, each additional sample of the multiple sample is run on the LC column. In embodiments, the samples may be run in sequence, for example as they are collected, or in a batch.

In embodiments, high-performance liquid chromatography (HPLC) is used. The primary difference between HPLC and conventional LC lies in the pressure applied during the process. HPLC utilizes higher pressures (up to several hundred atmospheres) to force the mobile phase through a tightly packed column, resulting in faster and more efficient separation of components. This high pressure allows for the use of smaller particle sizes in the stationary phase, which leads to sharper peaks and improved resolution of closely related compounds.

In embodiments, the LC column characterized by being a silica column with C18 bonding and a Lewis acid/Lewis base endcapping. Silica has a high surface area, which provides good interaction between the stationary phase and analytes, leading to efficient separations. C18 refers to the bonding of an 18-carbon-long alkyl chain (octadecyl group) to the silica surface. The C18 bond is typically very stable, providing good mechanical and chemical stability. In endcapped columns, the free silanol groups on the silica surface are chemically neutralized. Lewis acids are electron-pair acceptors, while Lewis bases are electron-pair donors, and both types of interactions help reduce undesirable interactions with polar or basic analytes, which can lead to peak tailing or poor reproducibility. By reducing such interactions, Lewis acid/Lewis base endcapping improves column performance, providing better peak symmetry and more stable retention times.

In embodiments, the LC column is further characterized by a first mobile phase and a second mobile phase. Use of multiple mobile phases can optimize the separation of analytes with different affinities for the stationary phase. In embodiments, the first mobile phase may be Triethylammonium bicarbonate (TEAB). TEAB provides a stable pH environment and maintains the ionization states of analytes, which contributes to reproducible retention times and optimal separation. As a mobile phase component, TEAB is useful in gradient elution to enhance analyte solubility and to prevent unwanted interactions with the stationary phase. In embodiments, the first mobile phase may be at a 25 mM concentration.

In embodiments, the second mobile phase may be Acetonitrile (ACN). ACN is characterized by its low viscosity, high solvent strength, and ability to solvate a wide range of analytes. It enhances the elution of hydrophobic analytes from the stationary phase (e.g., C18), allowing for better resolution of compounds with varying polarities. Additionally, ACN has a low UV absorbance, which minimizes interference during detection.

In gradient elution, the concentration of ACN is gradually increased, aiding the separation of both hydrophilic and hydrophobic compounds. The gradient profile, including the slope (rate of change) and whether it is linear or nonlinear, directly impacts separation efficiency. A steeper slope speeds up the separation but may reduce resolution, while a slower gradient improves resolution but increases run time. The initial and final mobile phase compositions must be tailored to the nature of the analytes. Balancing these compositions helps ensure the best separation of compounds based on their polarity. For example, in some preferred embodiments the second mobile phase is ramped from a 15% initial concentration a 100% final concentration. In some examples, the ramping is performed over a period of about 2 min, about 3 min, about 5 min, about 7 min, about 9 min, about 10 min, etc.

At operation 206, a calibration curve is generated. In embodiments, the calibration curve may be a first calibration curve of one or more calibration curves. For example, the first calibration curve is configured to have an R² value of at least 0.998 for all nucleotides.

Calibration curves are generally generated based on a series of standard solutions with known concentrations of the analyte(s) of interest. These standards should span the expected concentration range for the analyte in the samples being tested. Once the standard solutions are prepared, each is injected into the HPLC system under the same conditions that will be used for analyzing the unknown samples. The retention time and peak area (or peak height, depending on the method) of each standard are recorded. The peak area is generally preferred for quantification as it is less affected by variations in detector response. The concentration of each standard is plotted against the corresponding peak area or height to generate the calibration curve.

After plotting the data, a best-fit line (e.g., a linear regression) is applied to the data points, resulting in the calibration curve. The equation of this line (often expressed as y = mx + b, where y is the peak area, m is the slope, x is the concentration, and b is the y-intercept) can then be used to calculate the concentration of analytes in unknown samples based on their peak areas. The linearity of the calibration curve can be assessed by evaluating the correlation coefficient (R²) to ensure it's close to 1, indicating a strong relationship between concentration and peak area. Accuracy of the curve can be verified by analyzing quality control (QC) samples with known concentrations and ensuring that the results are consistent with the expected values.

At operation 208, one or more peaks are quantitated against a calibration curve. In embodiments where multiple samples are drawn and run on the LC column, one or more peak may be quantitated for each sample run. Quantifying peaks is generally performed by measuring the area under the curve or the peak height in the chromatogram. The area may be preferred as providing a more accurate representation of the amount of analyte present, as it is less affected by variations in peak shape or detector response.

Each peak corresponds to a specific component in the sample, and the area under each peak can be calculated using software integrated with the HPLC system. For more accurate quantification, the peak area is compared to a calibration curve generated from known concentrations of a standard analyte. By plotting the peak area against the concentration of the standard solutions, a linear relationship is established, and the concentration of unknown analytes in the sample can be determined based on their peak areas. In embodiments, at least one quantitated peak is selected from a peak for CTP, a peak for ATP, a peak for UTP, a peak for GTP, a peak for RNA, a peak for plasmid DNA, and a peak for a cap analog. Advantageously, at least the peak for CTP and the peak for UTP do not overlap with each other. In embodiments, each of the peak for CTP, the peak for ATP, the peak for UTP, the peak for GTP, the peak for RNA and the peak for a cap analog do not overlap with any other peak.

In embodiments, the result from quantitating the one or more peaks against a first calibration curve is used to determine degradation or contamination in the IVT process. The RNA product is expected to produce a single, well-defined peak corresponding to the expected RNA size. However, if degradation occurs, it often results in the formation of smaller RNA fragments, which will elute at different retention times during HPLC analysis. These smaller peaks, or a broader distribution of peaks, can be identified and quantified to assess the extent of degradation. The area under these peaks can be compared to the area of the main RNA peak, with a higher ratio indicating more significant degradation.

Contamination can also be detected by quantifying peaks that correspond to unwanted substances, such as residual nucleotides (NTPs), polymerase, or salts. These contaminants will produce distinct peaks in the chromatogram, separate from the RNA product. By comparing the intensity of these peaks to the intensity of the RNA peak, the level of contamination can be determined. For example, unreacted NTPs may show a separate peak at the characteristic retention time of the individual nucleotides, and a high intensity of these peaks suggests incomplete transcription or poor reaction efficiency. A clean, un-degraded RNA sample should have a sharp, distinct peak, with minimal or no interference from contaminants. If contamination or degradation is detected, adjustments in reaction conditions (e.g., using RNase inhibitors, optimizing reagent quality) may be introduced to improve the yield and purity of the RNA product.

At operation 210, an RNA product is quantified. In embodiments, the RNA produced is quantified by comparison to a DNA-only standard. For example, the DNA-only standard is run and a DNA-only concentration is determined based on the run. The DNA-only concentration is then subtracted from a mixed RNA/DNA concentration peak.

In embodiments where the fed batch IVT process is used, one or more additional reagents are added during the course of the IVT process based on at least one quantitated peak, as at operation 212. In embodiments, the additional reagents are based on at least two quantitated peaks, based on at least three quantitated peaks, based on at least four quantitated peaks, based on at least five quantitated peaks, based on at least six quantitated peaks, based on seven quantitated peaks, etc. In some instances, the one or more additional reagents added to the IVT process improve yield of RNA, decrease use of additional reagents, or both.

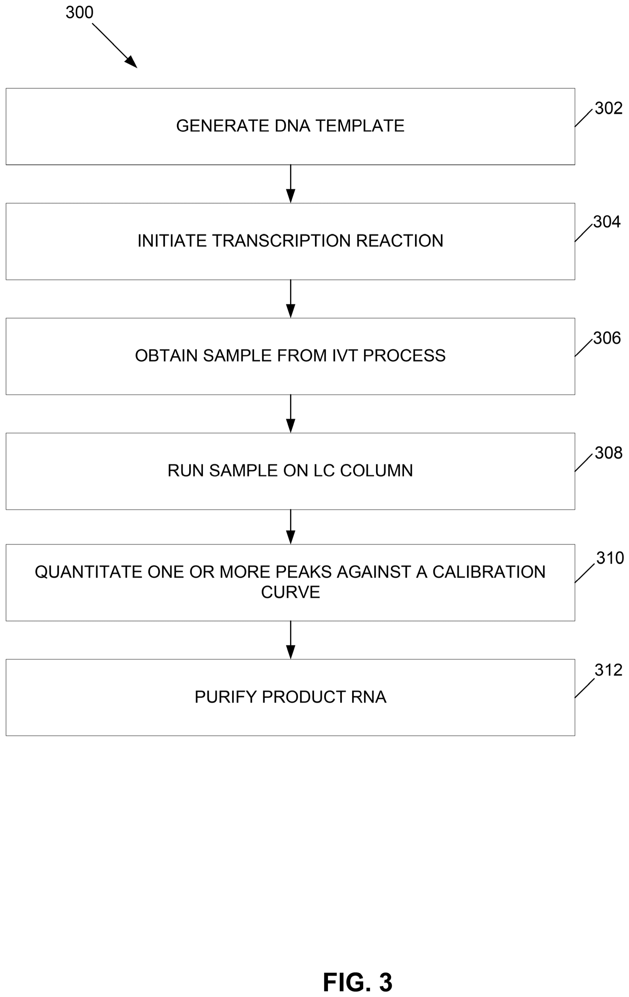

FIG. 3 is a flowchart of an example method 300 of in vitro transcription.

At operation 302, a DNA template is generated. The DNA template, starts with the gene of interest, often cloned into a plasmid vector, which contains the promoter sequence and any additional necessary elements, such as restriction enzyme sites, selection markers, or polyadenylation signals. The plasmid construct is then amplified isolated from the amplification host (usually by a plasmid extraction kit). Before proceeding with IVT, the plasmid may be linearized to create a DNA template that can be efficiently transcribed.

At operation 304, a transcription reaction is initiated including one or more reagents. Once the DNA template is prepared and confirmed to be of high quality, it is mixed with the necessary reagents for the IVT reaction, including NTPs, RNA polymerase, and the appropriate buffer system. The linearized DNA template serves as the starting point for RNA synthesis, and the reaction conditions are optimized to produce a high yield of the desired RNA product.

Method 300 proceeds with monitoring the transcription reaction as discussed with respect to operations 306-312. At operation 306, a sample is obtained from an IVT process at a timepoint. At operation 308, the sample is run on a liquid chromatography (LC) column. In embodiments, the LC column characterized by being a silica column with C18 bonding and a Lewis acid/Lewis base endcapping. At operation 310, one or more peaks are quantified against a calibration curve. Operations 306-310 substantially coincide with steps 202, 204, and 208 of method 200 of FIG. 2.

At operation 312, a product RNA is purified.

Purifying RNA products after IVT reaction ensures the removal of any residual reagents, unincorporated NTPs, DNA template, enzymes, or other contaminants that could interfere with downstream applications. The purification process isolates the desired RNA while maintaining its integrity and functionality.

Steps in include treating the reaction mixture with a DNAse I enzyme to degrade an lingering DNA template. The reaction may be incubated for a short time to ensure complete degradation of the DNA. The RNA product can then be separated from the DNase and other reaction components by methods such as phenol-chloroform extraction or spin-column-based purification. These methods effectively remove contaminants while retaining the RNA in an aqueous phase. The RNA may be concentrated and further purified by techniques such as gel electrophoresis or silica-column-based RNA purification. For applications requiring extremely pure RNA, additional steps like RNA precipitation using ethanol or isopropanol can help remove trace impurities.

### Examples

### Example 1. Experimental conditions.

The following examples demonstrate data obtained in particular use-case as proof of concept. The instrument, mobile phase, and gradient used in the following examples is summarized in Tables 1 and 2.

**Table 1. Summary of experimental conditions for Examples.**

| | |
|---|---|
| Instrument | Thermo Dionex UltiMate 3000 or equivalent |
| Mobile Phase A | *25 mM Triethylammonium bicarbonate (TEAB), pH 7 +/- 0.5 |
| Mobile Phase B | 100% Acetonitrile (ACN) |
| Column | YMC-Pack Pro C18, 250 x 4.6 mm, 5 um, 120 A |
| Gradient | Ramp to 15% B, see Table 2 |
| Autosampler Temp | 10 C |
| Column Temp | 25 C |
| Flow Rate | 1.0 mL/m |
| Injection Volume | 10 uL |
| Run Time | 63 m (51 m + 12 m equilibration) |

**Table 2. Details of Gradient from Table 1.**

| **Time (m)** | **%A** | **%B** |
|---|---|---|
| **-12** | 100 | 0 |
| **0** | | INJECT |
| **7** | 97.5 | 2.5 |
| **21** | 93 | 7 |
| **25** | 88.5 | 11.5 |
| **32** | 85 | 15 |
| **39** | 0 | 100 |
| **51** | | STOP |

### Example 2. NTP calibration curves.

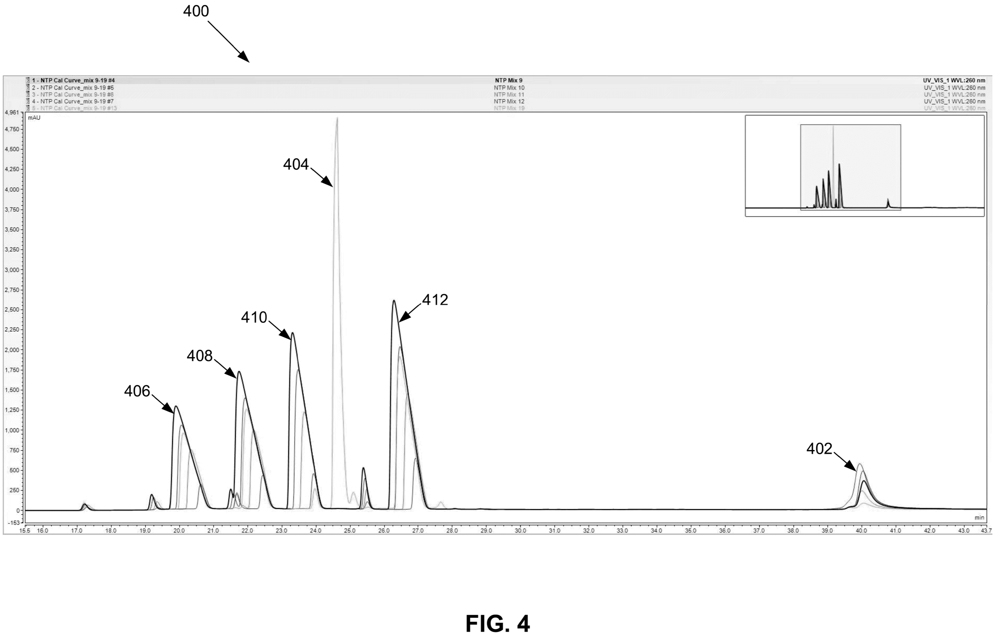

FIG. 4 is an example chromatogram 400 with multiple runs of IVT reagents for generating NTP calibration curves. Individual peaks are grouped for the key regents and product, including an mRNA group 402, a cap reagent group 404, a CTP group 406, a UTP group 408, a GTP group 410, and an ATP group 412. As may be discerned by the variation among the peaks in each group, the multiple runs used to generate example chromatogram 400 included varying amounts of the reagents, which variation among the amount of output product, mRNA group 402, resulting. Measurements from the samples used to generate chromatogram 400 are summarized in Table 3.

**Table 3. Sample mixes contributing to chromatogram 400 of FIG. 4, measured at UV 260 nm.**

| Mix no. | CTP | UTP | GTP | ATP | ARCA | mRNA |
|---|---|---|---|---|---|---|
| 9 | 785.98 | 1046.73 | 1165.82 | 1486.34 | 0 | 184.22 |
| 10 | 553.55 | 724.13 | 801.68 | 1014.89 | 0 | 251.5 |
| 11 | 296.06 | 383.32 | 420.87 | 566.60 | 0 | 125.41 |
| 12 | 85.1 | 104.97 | 88.55 | 151.82 | 0 | 339.79 |
| 19 | 443.03 | 576.15 | 38.87 | 884.71 | 1096.19 | 54.1 carryover (none expected) |

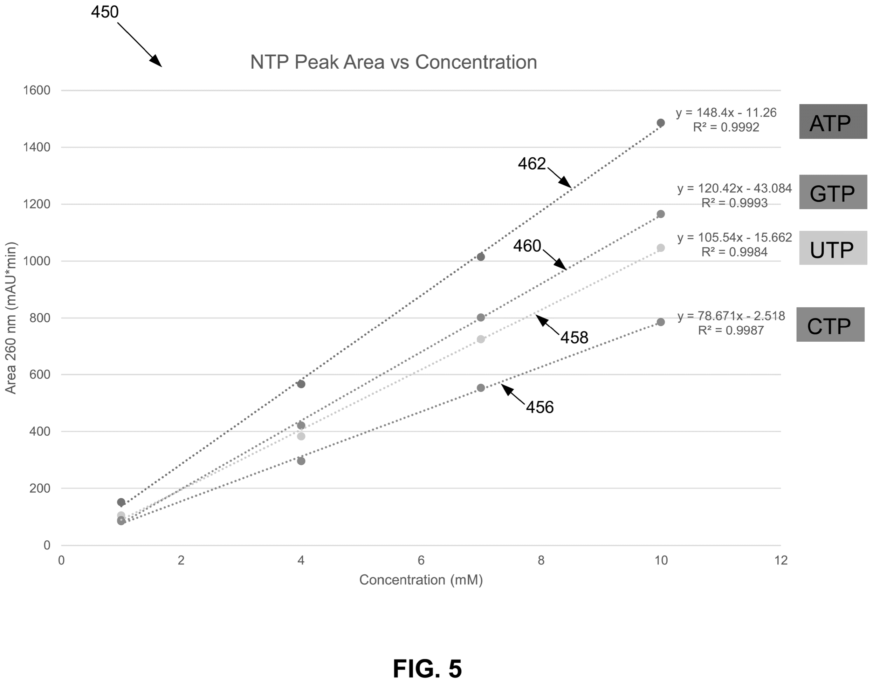

FIG. 5 is an example graph 450 of calibration curves 456-462 generated using data from chromatogram 400 of FIG. 4. A calibration curve is generated for each of the NTPs, with a CTP curve 456, a UTP curve 458, a GTP curve 460, and an ATP curve 462. Each of the NTP calibration curves 456-462 is at least a four-point curve based on data from mixes of varying concentrations of NTPs. In this example, each of the calibration curves has an R² value of greater than 0.998.

### Example 3. Cap reagent calibration curve.

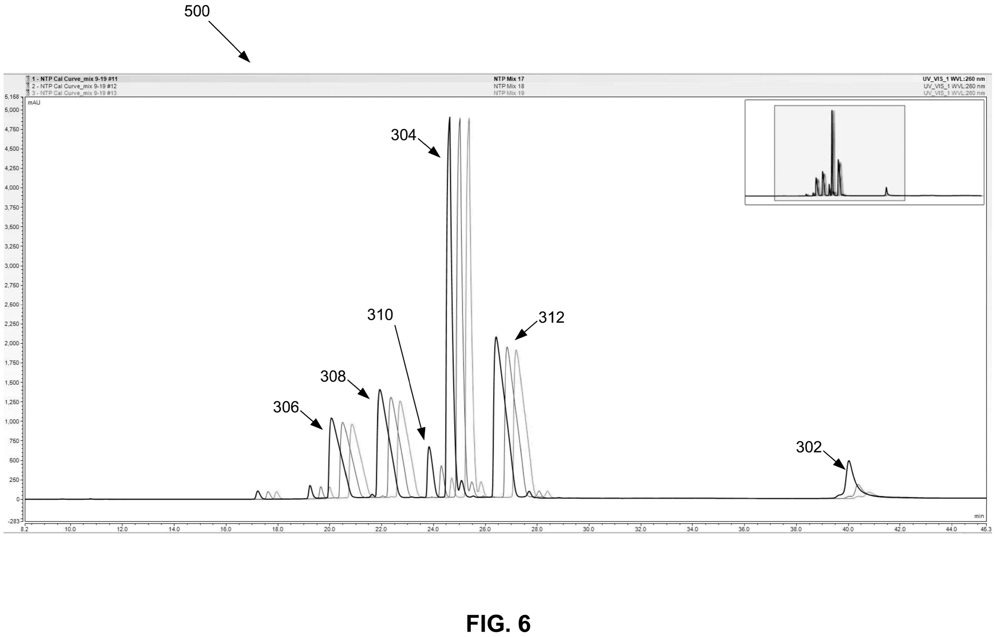

FIG. 6 is an example chromatogram 500 with multiple runs of IVT reagents for generating cap reagent and mRNA product calibration curves. Individual peaks are grouped for the key regents and product, including an mRNA group 302, a cap reagent group 304, a CTP group 306, a UTP group 308, a GTP group 310, and an ATP group 312. As may be discerned by the variation among the peaks in each group, the multiple runs used to generate example chromatogram 500 included varying amounts of the reagents, which differ from the multiple runs used to generate chromatogram 400 of FIG. 4 and are summarized in Table 4 below.

**Table 4. Sample mixes contributing to chromatogram 500 of FIG. 6, measured at UV 260 nm.**

| Mix no. | CTP | UTP | GTP | ATP | ARCA | mRNA |
|---|---|---|---|---|---|---|
| 17 | 523.85 | 688.02 | 150.96 | 1032.46 | 1147.9 | 242.15 carryover (none expected) |
| 18 | 473.05 | 621.68 | 78.02 | 928.16 | 1139.69 | 94.6 carryover (none expected) |
| 19 | 443.03 | 576.15 | 38.87 | 884.71 | 1096.19 | 54.1 carryover (none expected) |

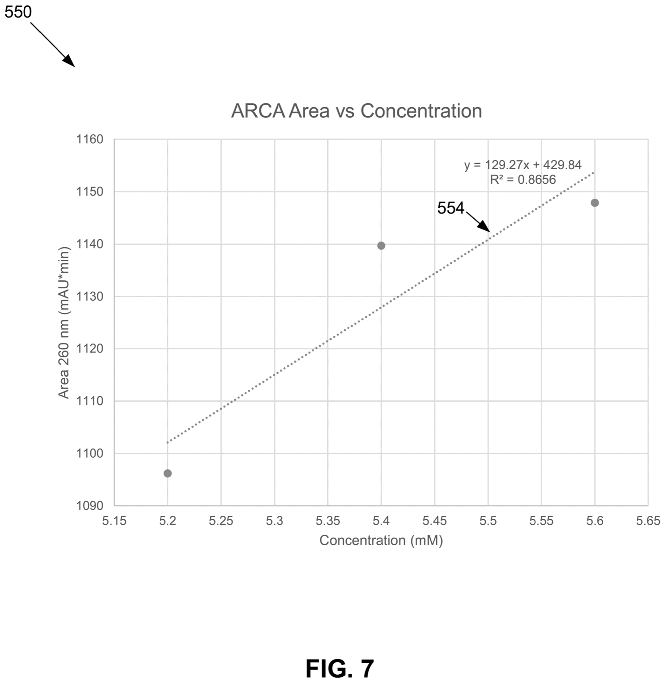

FIG. 7 is an example graph 550 of a calibration curves 554 generated using data from chromatogram 500 of FIG. 5. Calibration curve 554 is generated for the cap reagent, ARCA in this example, using three data points from chromatogram 500 of FIG. 5.

### Example 4. mRNA calibration curve.

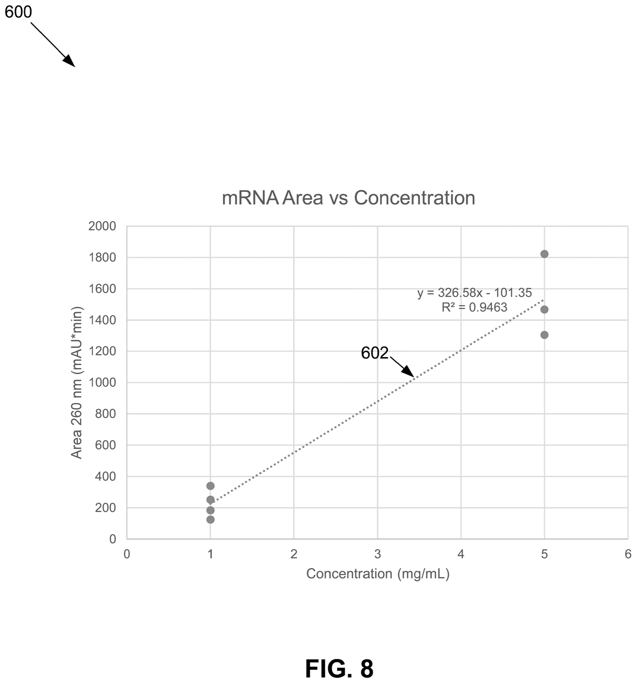

FIG. 8 is an example graph 600 of a calibration curve 602 for mRNA. The example calibration curve 602 was generate using the data summarized in Table 5.

**Table 5. Sample mixes used to generate example mRNA calibration curve.**

| Mix no. | CTP | UTP | GTP | ATP | ARCA | mRNA |
|---|---|---|---|---|---|---|
| 9 | 785.98 | 1046.73 | 1165.82 | 1486.34 | 0 | 184.22 |
| 10 | 553.55 | 724.13 | 801.68 | 1014.89 | 0 | 251.5 |
| 11 | 296.06 | 383.32 | 420.87 | 566.60 | 0 | 125.41 |
| 12 | 85.1 | 104.97 | 88.55 | 151.82 | 0 | 339.79 |
| 13 | 776.97 | 1037.25 | 1182.29 | 1508.5 | 0 | 1303.95 |
| 14 | 528.54 | 702.56 | 805.06 | 1115.25 | 0 | 1468.2 |
| 15 | 282.7 | 358.71 | 365.16 | 539.88 | 0 | 1822.5 |

### Example 5. Applying calibration curves to sample run.

Table 6 summarizes results evaluating the calibration curves generated in Examples 3-5 in application to a sample.

**Table 6. Results and conclusions from application of NTP calibration curves 456-462 from FIG. 5, cap reagent calibration curve 554 from FIG. 7, and mRNA calibration curve 602 from FIG. 8 to a test sample.**

| | Observed area | Conc. (mM) determined | Conc. (mM) expected | % error |
|---|---|---|---|---|
| CTP | 563.43 | 7.19 | 7.5 | 4.08 |
| UTP | 751.69 | 7.27 | 7.5 | 3.06 |
| GTP | 900.58 | 7.84 | 7.5 | 4.49 |
| ATP | 1149.49 | 7.82 | 7.5 | 4.29 |
| ARCA | 1078.92 | 5.02 | 5 | 0.42 |
| mRNA | 707.18 | 2.48 | 3.75 | 33.98 |

Despite the high error rate for mRNA, which was expected due to noted carryover of RNA, all reagents satisfactorily presented with less than 5% deviation from expected values.

### Example 6. Applying calibration curves to crude IVT sample.

Table 7 summarizes results evaluating the calibration curves generated in Examples 3-5 in application to a crude IVT sample.

**Table 7. Results and conclusions from application of NTP calibration curves 456-462 from FIG. 5, cap reagent calibration curve 554 from FIG. 7, and mRNA calibration curve 602 from FIG. 8 to a crude IVT sample.**

| | Observed area | Conc. (mM) determined |
|---|---|---|
| CTP | 546.57 | 6.98 |
| UTP | 744.49 | 7.20 |
| GTP | 757.27 | 6.65 |
| ATP | 1024.29 | 6.98 |
| mRNA/pDNA | 884.47 | 3.02 |

The results of Table 7 suggest NTPs are all about 7 mM and mRNA and pDNA, which are noted to elute together, are about 3 mg/mL. Results indicate that the developed method can be run on crude IVT with no observable interference.

### Example 7. Gradient optimization.

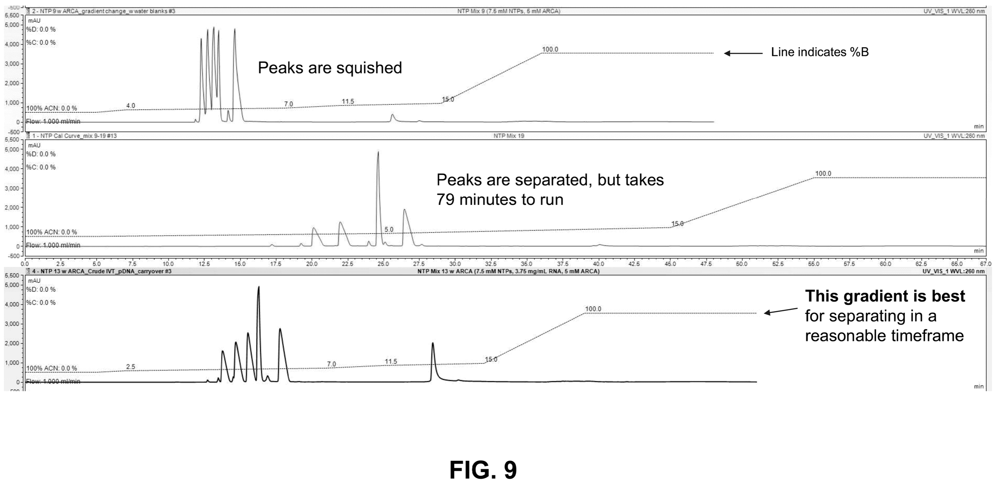

FIG. 9 is a comparison of three gradients demonstrating an example preferred gradient for provided adequate separation in a desired timeframe.

Illustrative examples of the systems and methods described herein are provided below. An embodiment of the system or method described herein may include any one or more, and any combination of, the clauses described below.

Clause 1. A method of monitoring an in vitro transcription (IVT) process, the method including: obtaining a sample from an IVT process at a first timepoint; running the sample on a liquid chromatography (LC) column, the LC column characterized by being a silica column with C18 bonding and a Lewis acid/Lewis base endcapping; and quantitating one or more peaks against a first calibration curve.

Clause 2. The method of clause 1, wherein the LC column is further characterized by a first mobile phase and a second mobile phase.

Clause 3. The method of clause 2, wherein the first mobile phase is Triethylammonium bicarbonate (TEAB).

Clause 4. The method of clause 3, wherein the first mobile phase is at a 25 mM concentration.

Clause 5. The method of any one of clauses 2-4, wherein the second mobile phase is Acetonitrile (ACN).

Clause 6. The method of clause 5, wherein the second mobile phase is ramped from a 15% initial concentration a 100% final concentration.

Clause 7. The method of clause 1, further comprising generating the first calibration curve.

Clause 8. The method of clause 7, wherein the first calibration curve is configured to have an R² value of at least 0.998 for all nucleotides.

Clause 9. The method of clause 1, wherein the sample is one of a clean sample or a crude sample.

Clause 10. The method of clause 1, further including quantifying an RNA product by: running a DNA-only standard; determining a DNA-only concentration; subtracting the DNA-only concentration from a mixed RNA/DNA concentration peak of the one or more peaks.

Clause 11. The method of clause 1, wherein the IVT process is one of a fed batch IVT process and a simple IVT process.

Clause 12. The method of clause 11 , wherein the fed batch IVT process is used, and one or more additional reagents are added to the IVT process based on at least one quantitated peak, based on at least two quantitated peaks, based on at least three quantitated peaks, based on at least four quantitated peaks, based on at least five quantitated peaks, based on at least six quantitated peaks, or based on seven quantitated peaks.

Clause 13. The method of clause 12, wherein the at least one quantitated peak is selected from a peak for CTP, a peak for ATP, a peak for UTP, a peak for GTP, a peak for RNA, a peak for plasmid DNA, and a peak for a cap analog.

Clause 14. The method of clause 13, wherein at least the peak for CTP and the peak for UTP do not overlap with each other.

Clause 15. The method of clause 14, wherein the peak for CTP, the peak for ATP, the peak for UTP, the peak for GTP, the peak for RNA and the peak for a cap analog do not overlap with any other peak.

Clause 16. The method of any one of clauses 12-15, wherein the one or more additional reagents added to the IVT process improve yield of RNA, decrease use of additional reagents, or both.

Clause 17. The method of clause 1, wherein high-performance liquid chromatography is used.

Clause 18. The method of any one of clauses 1-17, further comprising obtaining a sample from the IVT process at additional timepoints; running each additional sample on a liquid chromatography (LC) column, the LC column characterized by being a silica column with C18 bonding and a Lewis acid/Lewis base endcapping; and quantitating one or more peaks against a first calibration curve.

Clause 19. The method of clause 18, further comprising adding one or more additional reagents to the IVT process based on at least one quantitated peak, based on at least two quantitated peaks, based on at least three quantitated peaks, based on at least four quantitated peaks, based on at least five quantitated peaks, based on at least six quantitated peaks, or based on seven quantitated peaks.

Clause 20. The method of clause 19, wherein the at least one quantitated peak is selected from a peak for CTP, a peak for ATP, a peak for UTP, a peak for GTP, a peak for RNA, a peak for plasmid DNA, and a peak for a cap analog.

Clause 21. The method of clause 20, wherein at least the peak for CTP and the peak for UTP do not overlap with each other.

Clause 22. The method of clause 21, wherein the peak for CTP, the peak for ATP, the peak for UTP, the peak for GTP, the peak for RNA and the peak for a cap analog do not overlap with any other peak.

Clause 23. The method of any one of clauses 19-22, wherein the one or more additional reagents added to the IVT process improve yield of RNA, decrease use of additional reagents, or both

Clause 24. The method of any one of clauses 1-23, wherein the quantitating one or more peaks against a first calibration curve is used to determine degradation or contamination in the IVT process.

Clause 25. A method of in vitro transcription, the method including: generating a DNA template; initiating a transcription reaction including one or more reagents; monitoring the transcription reaction by: obtaining a sample from an IVT process at a first timepoint; running the sample on a liquid chromatography (LC) column, the LC column characterized by being a silica column with C18 bonding and a Lewis acid/Lewis base endcapping; quantifying one or more peaks against a calibration curve; and purifying product RNA.

Clause 26. The method of clause 25, wherein the LC column is further characterized by a first mobile phase and a second mobile phase.

Clause 27. The method of clause 26, wherein the first mobile phase is Triethylammonium bicarbonate (TEAB).

Clause 28. The method of clause 27, wherein the first mobile phase is at a 25 mM concentration.

Clause 29. The method of any one of clause 26-28, wherein the second mobile phase is Acetonitrile (ACN).

Clause 30. The method of clause 29, wherein the second mobile phase is ramped from a 15% initial concentration a 100% final concentration.

Clause 31. The method of clause 25, further including generating the calibration curve.

Clause 32. The method of clause 31, wherein the calibration curve is configured to have an R² value of at least 0.998 for all nucleotides.

Clause 33. The method of clause 25, wherein the sample is one of a clean sample or a crude sample.

Clause 34. The method of clause 25, further including quantifying the product RNA by: running a DNA-only standard; determining a DNA-only concentration; subtracting the DNA-only concentration from a mixed RNA/DNA concentration peak of the one or more peaks.

Clause 35. The method of clause 25, wherein the IVT process is one of a fed batch IVT process and a simple IVT process.

Clause 36. The method of claim 35 , wherein the fed batch IVT process is used, and one or more additional reagents are added to the IVT process based on at least one quantitated peak, based on at least two quantitated peaks, based on at least three quantitated peaks, based on at least four quantitated peaks, based on at least five quantitated peaks, based on at least six quantitated peaks, or based on seven quantitated peaks.

Clause 37. The method of clause 36, wherein the at least one quantitated peak is selected from a peak for CTP, a peak for ATP, a peak for UTP, a peak for GTP, a peak for RNA, a peak for plasmid DNA, and a peak for a cap analog.

Clause 38. The method of clause 37, wherein at least the peak for CTP and the peak for UTP do not overlap with each other.

Clause 39. The method of clause 38, wherein the peak for CTP, the peak for ATP, the peak for UTP, the peak for GTP, the peak for RNA and the peak for a cap analog do not overlap with any other peak.

Clause 40. The method of any one of clauses 36-39, wherein the one or more additional reagents added to the IVT process improve yield of RNA, decrease use of additional reagents, or both.

Clause 41. The method of clause 25, wherein high-performance liquid chromatography is used.

Clause 42. The method of any one of clauses 25-41, further comprising obtaining a sample from the IVT process at additional timepoints; running each additional sample on a liquid chromatography (LC) column, the LC column characterized by being a silica column with C18 bonding and a Lewis acid/Lewis base endcapping; and quantitating one or more peaks against a first calibration curve.

Clause 43. The method of clause 42, further including adding one or more additional reagents to the IVT process based on at least one quantitated peak, based on at least two quantitated peaks, based on at least three quantitated peaks, based on at least four quantitated peaks, based on at least five quantitated peaks, based on at least six quantitated peaks, or based on seven quantitated peaks.

Clause 44. The method of clause 43, wherein the at least one quantitated peak is selected from a peak for CTP, a peak for ATP, a peak for UTP, a peak for GTP, a peak for RNA, a peak for plasmid DNA, and a peak for a cap analog.

Clause 45. The method of claim 44, wherein at least the peak for CTP and the peak for UTP do not overlap with each other.

Clause 46. The method of clause 45, wherein the peak for CTP, the peak for ATP, the peak for UTP, the peak for GTP, the peak for RNA and the peak for a cap analog do not overlap with any other peak.

Clause 47. The method of any one of clauses 43-46, wherein the one or more additional reagents added to the IVT process improve yield of RNA, decrease use of additional reagents, or both

Clause 48. The method of any one of clauses 25-47, wherein the quantitating one or more peaks against the calibration curve is used to determine degradation or contamination in the IVT process.

Having described the preferred aspects and implementations of the present disclosure, modifications and equivalents of the disclosed concepts may readily occur to one skilled in the art. However, it is intended that such modifications and equivalents be included within the scope of the claims which are appended hereto.

## Claims

1. A method of monitoring an in vitro transcription (IVT) process, the method comprising:
obtaining a sample from an IVT process at a first timepoint;
running the sample on a liquid chromatography (LC) column, the LC column **characterized by** being a silica column with C18 bonding and a Lewis acid/Lewis base endcapping; and
quantitating one or more peaks against a first calibration curve.

2. The method of claim 1, wherein the LC column is further **characterized by** a first mobile phase and a second mobile phase.

3. The method of claim 2, wherein the first mobile phase is Triethylammonium bicarbonate (TEAB).

4. The method of claim 3, wherein the first mobile phase is at a 25 mM concentration.

5. The method of any one of claims 2-4, wherein the second mobile phase is Acetonitrile (ACN).

6. The method of claim 5, wherein the second mobile phase is ramped from a 15% initial concentration to a 100% final concentration.

7. The method of claim 1, wherein a fed batch IVT process is used, and one or more additional reagents are added to the IVT process based on at least one quantitated peak, based on at least two quantitated peaks, based on at least three quantitated peaks, based on at least four quantitated peaks, based on at least five quantitated peaks, based on at least six quantitated peaks, or based on seven quantitated peaks.

8. The method of claim 7, wherein the at least one quantitated peak is selected from a peak for CTP, a peak for ATP, a peak for UTP, a peak for GTP, a peak for RNA, a peak for plasmid DNA, and a peak for a cap analog.

9. The method of any one of claims 1-8, further comprising obtaining a sample from the IVT process at additional timepoints;
running each additional sample on a liquid chromatography (LC) column, the LC column **characterized by** being a silica column with C18 bonding and a Lewis acid/Lewis base endcapping; and
quantitating one or more peaks against a first calibration curve.

10. A method of in vitro transcription, the method comprising:
generating a DNA template;
initiating a transcription reaction including one or more reagents;
monitoring the transcription reaction by:
obtaining a sample from an IVT process at a first timepoint;
running the sample on a liquid chromatography (LC) column, the LC column **characterized by** being a silica column with C18 bonding and a Lewis acid/Lewis base endcapping;
quantifying one or more peaks against a calibration curve; and
purifying product RNA.

11. The method of claim 10, wherein the LC column is further **characterized by** a first mobile phase and a second mobile phase.

12. The method of claim 11, wherein the first mobile phase is Triethylammonium bicarbonate (TEAB).

13. The method of claim 12, wherein the first mobile phase is at a 25 mM concentration.

14. The method of any one of claims 11-13, wherein the second mobile phase is Acetonitrile (ACN).

15. The method of claim 14, wherein the second mobile phase is ramped from a 15% initial concentration to a 100% final concentration.
